# EUROPÄISCHE PATENTSCHRIFT

(11) **EP 1 694 284 B1**
(45) Veröffentlichungstag und Bekanntmachung des Hinweises auf die Patenterteilung: **10.12.2008**
(21) Anmeldenummer: 03750663.1
(22) Anmeldetag: 30.09.2003
(51) Int. Cl.: A61K 8/18

(54) **KOSMETISCHES UND DERMATOLOGISCHES MITTEL MIT MAGNETTEILCHEN, DESSEN HERSTELLUNG UND VERWENDUNG**
COSMETIC AND DERMATOLOGICAL AGENT CONTAINING MAGNETIC PARTICLES, PRODUCTION THEREOF, AND USE OF THE SAME
AGENT COSMETIQUE ET DERMATOLOGIQUE CONTENANT DES PARTICULES MAGNETIQUES, FABRICATION ET UTILISATION

(43) Veröffentlichungstag der Anmeldung: 30.08.2006
(73) Patentinhaber: Coty B.V., 2031 CC Haarlem (NL)
(72) Erfinder: GOLZ-BERNER, Karin, 98000 Monaco (MC); ZASTROW, Leonhard, 98000 Monaco (MC)
(74) Vertreter: Ziebig, Marlene
(86) Internationale Anmeldenummer: PCT/EP2003/010847
(87) Internationale Veröffentlichungsnummer: WO 2005/041915

(56) Entgegenhaltungen:
- US-A- 5 800 835
- US-A- 5 961 988
- DATABASE WPI Section Ch, Week 200301 Derwent Publications Ltd., London, GB; Class B04, AN 2003-000282 XP002283254 & CN 1 363 282 A (YU B) 14. August 2002 (2002-08-14)

## Beschreibung

Die Erfindung betrifft ein kosmetisches und dermatologisches Mittel, das hartmagnetische Teilchen und weitere Wirkstoffe enthält sowie ein Herstellungsverfahren für dieses Mittel und die Verwendung.

Aus der DE-C-4325071 ist ein Präparat zur Durchblutungsförderung bekannt, das hartmagnetische Einbereichsteilchen mit einer Koerzitiv-Feldstärke von 3000 bis 5000 Oerstedt und mit Korngrößen im Bereich von 600 bis 1200 nm neben kosmetischen oder pharmazeutischen Trägerstoffen enthält. Darin genannt sind beispielsweise Barium- und Strontiumhexaferrite als Einbereichsteilchen, die insgesamt eine durchblutungsfördernde Wirkung ausüben.

Weiterhin ist aus der WO 98/44895 bekannt, daß die Wundheilungswirkung verstärkt werden kann, wenn hartmagnetische Teilchen von 100-550 nm zusammen mit sauerstoffbeladenen asymmetrischen lamellaren Aggregaten aus Phospholipiden und Fluorcarbonen eingesetzt werden.

Ferner ist aus der EP 236374 bekannt, kosmetische Zusammensetzungen mit einem Edelsteinpulver einer Korngröße 0,5-30 µm als allergenschwachen Farbstoff zu verwenden.

Der Erfindung liegt die Aufgabe zugrunde, die Durchblutungsförderung in der Haut und die Immunabwehr zu verbessern.

Erfindungsgemäß wird bereitgestellt ein kosmetisches und dermatologisches Mittel mit Magnetteilchen, umfassend einen Gehalt von
0,001 bis 2 Gew-% hartmagnetische Teilchen, die aus der Gruppe ausgewählt sind, die aus Bariumhexaferrit-Einkristallen, Strontium-hexaferrit-Einkristallen, Samarium-Cobalt-Teilchen (SmCo) und Neodym-Eisen-Bor-Teilchen (Nd₂Fe₁₄B mit jeweils einer Teilchengröße im Bereich von 80 bis 550 nm besteht, und wobei die Teilchen die eine Koerzitivkraft im Bereich von 80.000 bis 1.600.000 A/m haben;
0,0001 bis 0,05 Gew-% eines gemahlenen Jadesteines, bestehend im wesentlichen aus Jadeit, Nephrit oder einem Gemisch davon mit einer Teilchengröße im Bereich von 50 - 95 nm;
zusammen mit kosmetischen oder dermatologischen Hilfs-, Träger- oder weiteren Wirkstoffen oder einem Gemisch davon mit einem Anteil bis 100 Gew-%.

In einer Ausführungsform der Erfindung sind die hartmagnetische Teilchen verkapselt in wäßrigen Liposomen aus Phospholipiden und eingehüllt in ein Gel.

In einer weiteren vorteilhaften Ausführungsform können die hartmagnetischen Teilchen auch in asymmetrischen lamellaren Aggregaten eingelagert sein, wobei die asymmetrischen lamellaren Aggregate aus natürlichen Phospholipiden wie Lecithin und Fluorcarbonen bestehen und zusätzlich Alkohole, Wasser und einen Gelbilder enthalten. In diesen "Komplex" können auch die Jadeteilchen eingebracht sein.

In einer weiteren Ausführungsform der Erfindung können neben den Liposomen mit hartmagnetischen Teilchen oder asymmetrischen lamellaren Aggregaten mit hartmagnetischen Teilchen oder deren Gemischen zusätzlich asymmetrische lamellare Aggregate enthalten sein, wobei diese Aggregate, bestehend aus natürlichen Phospholipiden mit einem Phösphatidylcholingehalt von 10 bis 99 Gew-%, vorzugsweise 30-99 Gew-%, und Fluorcarbonen, mit Sauerstoff bis zur Sättigungsgrenze beladen sind.

Die Teilchengröße beträgt 80 - 550 nm für die hartmagnetischen Teilchen. Es können auch geringe Mengen größerer oder kleinerer Teilchen enthalten sein (unter 5 %), wesentlich ist jedoch, daß die mittlere Teilchengröße D₅₀ etwa 250 nm beträgt. Das bedeutet, daß wenigstens 50 % der in einer Emulsion enthaltenen hartmagnetischen Teilchen in der Größenordnung 250 µm liegen. Eine typische Teilchengrößenverteilung ist beispielsweise

| | |
|---|---|
| 15 % | 80 bis 100 nm |
| 55 % | 100 bis 250 nm |
| 30 % | 250 bis 350 nm. |

Eine bevorzugte Teilchengröße ist daher 80-350 nm.

Die eingesetzten Magnetteilchen sind Einkristalle mit einheitlicher magnetischer Orientierung. Besonders bevorzugt sind undotierte Barium- und Strontiumhexaferrite. Die Herstellung dieser Einkristalle erfolgt z.B. nach der Glaskristallisationstechnik durch Züchtung aus einer abgeschreckten Glasschmelze wie in DE 19715477 beschrieben. Geeignete Gläser sind z.B. solche aus 20-50 Gew-% Fe₂O₃, 30-50 Gew-% BaO und 20-50 Gew-% B₂O₃.

Es wurde überraschen gefunden, daß der Zusatz von 0,0001 bis 0,05 Gew-% fein gemahlenen Jadesteines zu den hartmagnetischen Teilchen den Funktionszustand der Mikrozirkulation deutlich über dem zu erwartenden Wert erhöht, bezogen auf den Vergleichswert ohne den Zusatz. Die gute Mikrozirkulationswirkung der bekannten hartmagnetischen Teilchen in kosmetischen Zusammensetzungen wird damit deutlich übertroffen und damit der Antransport von Sauerstoff und Nährstoffen sowie der Abtransport von Stoffwechsel-Endprodukten im Hautgewebe nochmals verbessert.

Ebenso wurde die Änderung der lokalen Regelung der Mikrozirkulation deutlich über dem zu erwartenden Wert, bezogen auf den Vergleichswert ohne den Zusatz, erhöht. Die bereits durch die hartmagnetischen Teilchen allein vorgegebenen guten Werte werden gleichfalls noch einmal übertroffen und damit die Anpassungsbreite (Regelbreite) der Mikrozirkulation an sich ändernde Stoffwechselbedürfnisse der Haut entscheidend verbessert.

Es wurde weiterhin gefunden, daß die Randbedingungen für die immunologische Wirksamkeit der weißen Blutzellen als Hauptakteure der körpereigenen Abwehr in einem Ausmaß verbessert wurden, das außerordentlich überraschend ist. Gegenüber einer Vergleichssubstanz, die allein die hartmagnetischen Teilchen und Trägerstoffe enthält, erreichte ein erfindungsgemäßes kosmetisches Mittel bei gleicher Konzentration der hartmagnetischen Teilchen und einem Zusatz von nur 0,00025 Gew-% gemahlenem Jadestein, bezogen auf die Gesamtzusammensetzung, eine Erhöhung der Immunabwehr von nochmals 15-30 %.

Damit ist ein Nachweis für den Funktionszustand der Mikrozirkulation, deren Regelbreite und für den Zustand der Immunabwehr bei einem kosmetischen und dermatologischen Produkt gegeben, der für Prophylaxe und reale Anti-Alterungswirkung von anderen Produkten bisher nicht erreicht worden ist.

Der Jadestein liegt bevorzugt als Jadeit vor. Es kann auch ein Gemisch von Jadeit und Nephrit öder Nephrit allein eingesetzt werde.

Eine weitere Ausführungsform der Erfindung kann der Jadestein mit einem gemahlenen Malachitstein gleicher Korngröße im Verhältnis 1:0,2-3,5 vermischt sein, wobei nahezu gleiche Ergebnisse erhalten werden können.

Das erfindungsgemäße Kosmetikum oder Dermatikum enthält weiterhin übliche Hilfs- und Trägerstoffe, wie z.B. Wasser, Konservierungsmittel, Farbstoffe, Pigmente mit färbender Wirkung, Verdikungsmittel, Duftstoffe, Alkohole, Polyole, Ester, Elektrolyte, Gelbildner, polare und unpolare Öle, Polymere, Copolymere, Emulgatoren, Wachse, Stabilisatoren.

Es können auch weitere Wirkstoffe enthalten sein. Zu den kosmetischen Wirkstoffen gehören z. B. anorganische und organische Lichtschutzmittel, Radikalfänger, Feuchthaltemittel, Vitamine, Enzyme, pflanzliche Wirkstoffe, Polymere, Antioxidationsmittel, entzündungswidrige natürliche Wirkstoffe; Aufschlußprodukte von Hefen oder pflanzlichen Stoffen, hergestellt durch ein schonendes Ultraschall-Aufschlußverfahren gemäß WO 94/13783.

Als pharmakologisch besonders wirksame Substanzen sind besonders geeignet Heparin, Acetylsalicylsäure, Piroxicam, Miroxicam, Östrogene, Pseudohormone oder Phythohormone oder Gemische davon.

Die kosmetischen oder pharmakologisch wirksamen Substanzen können in der wäßrigen Phase der Emulsion vorhanden sein; sie können auch gesondert in Liposomen eingeschlossen sein.

Das erfindungsgemäße Mittel liegt vorteilhaft als Gel oder Emulsion oder Gel-Emulsion vor; letztere z.B. mit Emulgatoren, Wasser und einem Gel wie Carbopol.

Eine weitere Ausführungsform der Erfindung besteht darin, daß dem erfindungsgemäßen Gemisch von hartmagnetischen Einkristallen und gemahlenen Jadesteinen 0,1 bis 10 Gew-% eines kosmetisch annehmbaren Elektret-Feststoffes mit einer Teilchengröße von 0,05 bis 100 µm zugesetzt werden, wobei der Elektret ein induziertes permanentes Dipolmoment aufweist und ein permanentes elektrischen Feld mit einer Feldstärke von 500 bis 10⁷ Vm⁻¹ hat.

Bevorzugte Elektrete haben eine Feldstärke von 10⁵ bis 10⁷ Vm⁻¹.

Unter Elektreten werden allgemein Stoffe verstanden, die eine permanente entgegengesetzte elektrische Ladung an zwei gegenüberliegenden Flächen aufweisen. Damit bilden sie einen permanenten elektrischen Dipol, der von einem elektrischen Feld umgeben ist. Bestimmte natürliche Elektrete wie Turmalin sind bereits seit langem bekannt. Die Elektreteigenschaften können jedoch einer Reihe von Stoffen auch durch Einwirkung von außen verliehen werden. Dies kann thermisch erfolgen, indem der Stoff über die Curie-Temperatur erhitzt wird und in diesem Zustand einem elektrischen Feld ausgesetzt wird. Die sich im Feld orientierenden Dipole werden durch Abkühlung eingefroren.

Der dadurch erreichte Nichtgleichgewichtszustand geht nach einer bestimmten Relaxationszeit, die bei Elektreten im Bereich von Jahren liegt, wieder in einen Gleichgewichtszustand über. Die gleichen Zustände können auch photoelektrisch herbeigeführt werden.

Das Dipolmoment, das hier als "induziertes permanentes elektrisches Dipolmoment" bezeichnet werden soll, liegt deutlich über dem bekannten Dipolmoment von Stoffen, die nicht induziert worden sind.

Die Zerkleinerung des elektretischen Materials erfolgt für die kosmetische Anwendung nach der Induzierung bis zu einer Teilchengröße von 0,05 bis 100 µm, vorzugsweise 3 bis 80 µm.

Der Zusatz dieser Stoffe kann die Aufnahmefähigkeit der Hautzellen für Nähr- und Wirkstoffe deutlich verbessern, so daß die Hauptwirksamkeit der erfindungsgemäßen Mittel noch weiter erhöht werden kann.

Als Elektretmaterialien bevorzugt sind polymerisierte Fluorcarbone, die aus der Gruppe ausgewählt sind, bestehend aus Polytetrafluorethylen (PTFE), Fluorethylenpropylen (FEP), Polyvinylidenfluorid (PVDF), amorphes Fluorpolymer (AF) und als natürlicher Elektret gemahlener Turmalin sowie Gemische davon. PTFE ist besonders bevorzugt.

Gegenstand der Erfindung ist auch ein Verfahren zur Herstellung des kosmetischen oder dermatologischen Mittels mit Magnetteilchen. Das Verfahren besteht darin, daß zuerst ein Gemisch aus hartmagnetischen Teilchen, Jadeteilchen, einem oder mehreren Fluorcarbon(en), einem oder mehreren Phospholipid(en), Wasser, einem oder mehreren einwertigen und mehrwertigen Alkoholen und einem Gelbildner hergestellt wird, und das erhaltene Gel bei einer Temperatur im Bereich von 28-42°C mit weiteren kosmetischen Hilfs-, Träger- oder Wirkstoffen oder Gemischen davon vermischt wird, ohne die Temperatur über 42°C zu erhöhen.

Die Jadeteilchen bestehen bevorzugt aus Jadeit.

Als Phospholipide können z.B. eingesetzt werden Phosphatidylcholin, Phosphatidylethanolamin, Phosphatidylinositol, Phosphatidylserin, Phosphatidsäure und Lysolecithine sowie Gemische davon. Bekannte Produkte sind beispielsweise Phoslipon^{®} oder NAT^{®}. Es können jedoch auch äquivalente Stoffe wie Sphingolipide, wie z.B Sphingomyeline oder Ceramide eingesetzt werden.

Als einwertige Alkohole sind bevorzugt Ethanol und Isopropanol. Als mehrwertige Alkohole sind z.B Propylenglycol, Dipropylenglycol, Ethylenglycol, Isoprenglycol, Glycerin, Butylenglycole, Sorbitol und Gemische davon einsetzbar. Glycerin, Propylenglycol und Gemische davon sind bevorzugt.

Zu geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit.

Das erfindungsgemäß separat hergestellte Gemisch, das hier auch als "Komplex" bezeichnet ist, kann weitere Stoffe wie Konservierungsmittel, pH-Regulatoren wie Triethanolamin usw. enthalten.

Die Erfindung betrifft auch die topische kosmetische Verwendung eines Gemisches aus hartmagnetischen Einkristallen aus Barium- oder Strontiumhexaferrit der Teilchengröße 50-550 nm, wobei der Anteil 0,001-2 Gew-% beträgt, zusammen mit gemahlenem Jadestein der Teilchengröße 30-95 nm, wobei der Anteil Jade 0,0001 bis 0,05 Gew-% beträgt, sowie kosmetischen Hilfsstoffen, zur Erhöhung der Mikrozirkulation, der lokalen Regelung der Mikrozirkulation und der Immunabwehr auf Werte, die wenigstens 15 % über den Werten liegen, die ein Vergleichspräparat erreicht, das die gleiche Menge hartmagnetische Einkristalle allein enthält.

Weiterhin betrifft die Erfindung die topische dermatologische Verwendung eines Gemisches aus hartmagnetischen Einkristallen aus Barium- oder Strontiumhexaferrit der Teilchengröße 50-550 nm, wobei der Anteil >2 bis 6 Gew-% beträgt, zusammen mit gemahlenem Jadestein der Teilchengröße 30-95 nm, wobei der Jade-Anteil 0,05 bis 3 Gew-% beträgt, und dermatologischen Hilfsstoffen zur Herstellung eines Präparates zur Erhöhung der Mikrozirkulation, der lokalen Regelung der Mikrozirkulation und der Immunabwehr auf Werte, die wenigstens 20 % über den Werten liegen, die ein Vergleichspräparat erreicht, das die gleiche Menge hartmagnetische Einkristalle allein enthält.

Alle Prozentangaben sind auf das Gewicht der Gesamtzusammensetzung bezogen.

Die Erfindung soll nachstehend durch Beispiele näher erläutert werden. In der dazugehörigen Zeichnung zeigen
- Fig. 1:: Diagramm zur Verteilung der roten Blutzellen bei der Mikrozirkulation in der Haut;
- Fig. 2:: Diagramm zur immunologischen Widerstandsfähigkeit der Haut.

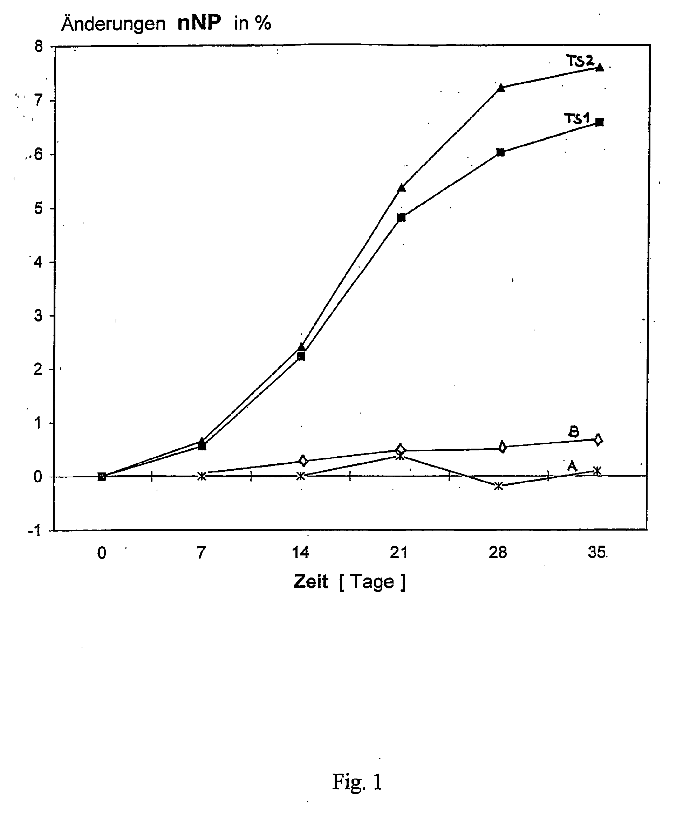

Fig. 1 stellt die prozentuale Verteilung der roten Blutzellen über die Zeit auf Basis der gemessenen Anzahl der aktuell blutzellperfundierten Knotenpunkte dar [nNP]; Ausgangswert n=60. Die blutzellperfundierten Knotenpunkte als Verzweigungsort im Mikrogefäß-Netzwerk widerspiegeln die Durchströmung der Kapillaren von Blutzellen. Die über 35 Tage untersuchte homogene Probanden-Stichprobe (18 Probandinnen, 35-45 Jahre, normaler Hauttyp) wurde im Doppelblindversuch in Abständen von 7 Tagen untersucht. Eingesetzt wurde eine Intravitalmikroskopische Untersuchungseinheit (OLYMPUS, Japan; KONTRON, USA) und eine Reflexionsspektrometrische Untersuchungseinheit (SPEX, USA). Die biometrische Analyse der gewonnenen Daten wurde mit dem WILCOXON-Rangsummentest auf dem Signifikanzniveau α=5% vorgenommen (zweiseitig).

Aus Fig. 1 ist zu entnehmen, daß beide Testsubstanzen TS1 und TS2 den Verteilungszustand des Blutes in der Haut-Mikrozirkulation in einem biologisch relevanten Ausmaß verbessern. Spätestens ab dem 14. Tag treten signifikante Merkmalsunterschiede auf (WILCOXON-Rangsummentest). Die Erhöhung bei der Testsubstanz TS2 liegt deutlich über der zu erwartenden additiven Erhöhung und zeigt somit in der Kombination hartmagnetische Teilchen plus Jadeteilchen einen Synergismus.

Auch der hier als Diagramm nicht dargestellte venuläre Abstrom aus den Netzwerken [Qven] zeigt ein gleichgerichtetes Merkmalverhalten, was durchaus bedeutungsvoll ist, da die meisten Störungen der Mikrozirkulation und damit auch die therapeutischen Konzepte den venulären Strömungsfluß betreffen. Ein gleiches Merkmalverhalten zeigt auch die lokale Regelbreite der Mikrozirkulation mit dem Merkmal "Flächeninhalt unter der Einhüllenden des Amplituden-Frequenz-Spektrums [AVM]"

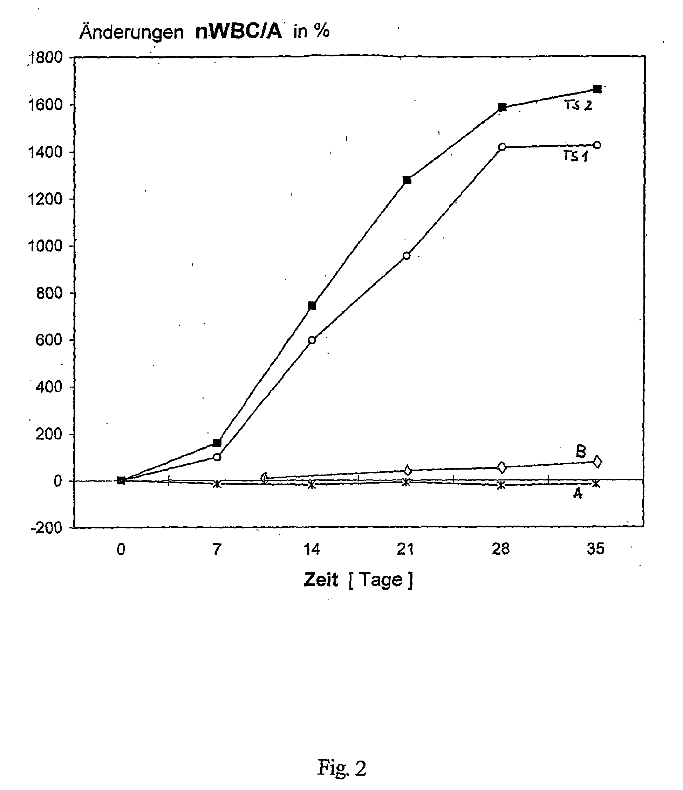

Fig. 2 stellt die prozentuale immunologische Widerstandfähigkeit der Haut auf Basis der gemessenen Anzahl der adhärierenden weißen Blutzellen an einer definierten Venolenwand dar [nWBC/A]. Die Steigerung über die Zeit läßt erkennen, daß die mikrohämodynamischen Randbedingungen für die Adhäsion der weißen Blutzellen am Venolen-Endothel verbessert werden und infolgedessen die Adhäsionen zunehmen. Die Untersuchungsbedingungen entsprachen den oben zu Fig. 1 genannten Bedingungen und wurden zeitgleich bei den Probandinnen am linken Unterarm auf unterschiedlichen Arealen der Hautoberfläche durchgeführt.

Aus Fig. 2 ist zu entnehmen, daß beide Testsubstanzen TS1 und TS2 die immunologische Widerstandsfähigkeit der Haut biologisch relevant verbessern, wobei die signifikanten Merkmalsunterschiede ab dem 14. Tag erkennbar sind (gemäß WILCOXON-Rangsummentest). Ebenso wie in Fig. 1 ist eine synergistische Wirksamkeit gegeben.

Die Testsubstanz TS1 entspricht dem Wirkkomplex gemäß Beispiel 1 ohne Jadezusatz und TS2 mit Jadezusatz. Kurve A ist der Kontrollwert bei einer unbehandelten Hautfläche, und Kurve B ist der Wert für den Wirkkomplex ohne Magnetteilchen aber mit Jadeteilchen.

Gegenüber bekannten Produkten zeigen die erfindungsgemäßen Mittel nachweislich eine nicht zu erwartende Steigerung der Mikrozirkulation, von deren Regelbreite und des Zustandes der Immunabwehr.

Die folgenden Angaben in den Beispielen sind in Gewichtsprozent, sofern nichts anderes angegeben ist.

### Beispiel 1 Tagescreme

| **Phase A** | |
|---|---|
| Wasser | q.s. ad 100 |
| Glycerin | 3 |
| Crosspolymer | 0,25 |

| **Phase B** | |
|---|---|
| Steareth-21 | 2,1 |
| Steareth-2 | 1,35 |
| Stearyl Alcohol | 0,5 |
| Dimethicone | 4,0 |
| Macadamia seed oil | 2,0 |

| **Phase C** | |
|---|---|
| Triethanolamin (TEA) | 0,25 |

| **Phase D** | |
|---|---|
| Wirkkomplex^{*} | 0,05 |
| Parfüm | 0,5 |
| Konservierungsmittel | 0,3 |

| | |
|---|---|
| ^{*} 6 % Bariumhexaferritteilchen D₅₀=230 nm, 160.000-280.000 A/m; 0,5 % Jadeteilchen D₅₀=75 nm; 2 % Perfluordecalin; 5 % Phospholipide; 2 % Propylenglycol; 10 % Glycerin; 1 % Carbomer; 3 % Ethanol; 0,1 % TEA; 70,4 % Wasser. | |

Die Phasen A und wurden separat hergestellt und bei 70°C unter Rühren zusammengeführt und vermischt. Die Phase C wurde bei 40°C zugegeben, und die separat hergestellte Phase D wurde bei 35°C unter Rühren zu dem Gemisch gegeben.

### Beispiel 2 Nachtcreme

| | |
|---|---|
| Wasser | q.s. ad 100 |
| Glycerin | 3 |
| Crosspolymer | 1,5 |

| **Phase B** | |
|---|---|
| Shea butter Fruit | 10 |
| Petrolatum | 4 |
| Silicone | 5,0 |

| **Phase C** | |
|---|---|
| Triethanolamin (TEA) | 1,5 |

| **Phase D** | |
|---|---|
| Wirkkomplex^{*} | 0,5 |
| Parfüm | 0,5 |
| Konservierungsmittel | 0,5 |

| | |
|---|---|
| ^{*} wie Beispiel 1, jedoch 25 % Bariumexaferritteilchen und 1 % Jadeteilchen | |

### Beispiel 3 Kosmetische Maske

| **Phase A** | |
|---|---|
| Wasser | q.s. ad 100 |
| Glycerin | 5,0 |
| Crosspolymer | 1,0 |

| **Phase B** | |
|---|---|
| Petrolatum | 10,0 |
| Silicone | 8,0 |

| **Phase C** | |
|---|---|
| TEA | 1,0 |

| **Phase D** | |
|---|---|
| Wirkkomplex ^{*} | 0,8 |
| Teflon^{®} (PTFE) | 1,5 |
| Parfüm | 0,5 |
| Konservierungsmittel | 0,5 |

| | |
|---|---|
| * wie Beispiel 1, jedoch 1 % Bariumexaferritteilchen und 0,5 % Jadeteilchen; Wasser 75,4 %. | |

## Patentansprüche

1. Kosmetisches und dermatologisches Mittel mit Magnetteilchen, umfassend einen Gehalt von 0,0001 bis 2 Gew-% hartmagnetische Teilchen, die aus der Gruppe ausgewählt sind, die aus Bariumhexaferrit-Einkristallen, Strontium-hexaferrit-Einkristallen, Samarium-Cobalt-Teilchen (SmCo) und Neodym-Eisen-Bor-Teilchen (Nd₂Fe₁₄B) mit jeweils einer Teilchengröße im Bereich von 80 bis 550 nm besteht,
und einer Koerzitivkraft der Teilchen im Bereich von 80.000 bis 1.600.000 A/m;
und 0,0001 bis 0,05 Gew-% eines gemahlenen Jadesteines mit einer Teilchengröße im Bereich von 50 - 95 nm
zusammen mit kosmetischen oder dermatologischen Hilfs- und Trägerstoffen bis 100 Gew-%.

2. Mittel nach Anspruch 1, **dadurch gekennzeichnet, daß** die hartmagnetischen Teilchen in Liposomen, asymmetrischen lamellaren Aggregaten oder Gemischen davon in einem Gel eingelagert sind.

3. Mittel nach Anspruch 1, **dadurch gekennzeichnet, daß** zusätzlich asymmetrischen lamellare Aggregate, beladen mit Sauerstoff enthalten sind.

4. Mittel nach Anspruch 1, **dadurch gekennzeichnet, daß** der Anteil an Jadestein im Bereich von 0,002 bis 0,02 Gew-% liegt.

5. Mittel nach Anspruch 1, **dadurch gekennzeichnet, daß** es weiterhin einen Gehalt an 0,1 bis 10 Gew-% eines kosmetisch annehmbaren Elektret-Feststoffes mit einer Teilchengröße von 0,05 bis 100 µm hat, wobei der Elektret ein induziertes permanentes Dipolmoment aufweist und ein permanentes elektrisches Feld mit einer Feldstärke von 500 bis 10⁷ Vm⁻¹ hat.

6. Mittel nach Anspruch 5, **dadurch gekennzeichnet, daß** der Elektret Polytetrafluorethylen (PTFE), Fluorethylenpropylen, Polyvinylidenfluorid, amorphes Fluorpolymer, Turmalin oder ein Gemisch davon ist, vorzugsweise PTFE.

7. Mittel nach Anspruch 1, **dadurch gekennzeichnet, daß** es ein Gemisch von Jade und Malachit enthält.

8. Verfahren zur Herstellung eines kosmetischen oder dermatologischen Mittels mit Magnetteilchen nach Anspruch 1,
**dadurch gekennzeichnet, daß** ein Gemisch aus hartmagnetischen Teilchen, Jadeteilchen, einem oder mehreren Fluorcarbon(en), einem oder mehreren Phospholipid(en), Wasser, einem oder mehreren einwertigen und mehrwertigen Alkoholen und einem Gelbildner hergestellt wird, und das erhaltene Gel bei einer Temperatur im Bereich von 28-42 °C mit weiteren kosmetischen Hilfs-, Träger- oder Wirkstoffen oder Gemischen davon vermischt wird, ohne die Temperatur über 42 °C zu erhöhen.

9. Topische Verwendung eines Gemisches aus hartmagnetischen Einkristallen aus Barium- oder Strontiumhexaferrit der Teilchengröße 50-550 nm, wobei der Anteil 0,001 bis 2 Gew-% beträgt und einer Koerzitivkraft der Teilchen im Bereich von 80.000 bis 1.600.000 A/m, zusammen mit gemahlenem Jadestein der Teilchengröße 50-95 nm, wobei der Anteil Jade 0,0001 bis 0,05 Gew-% beträgt, und kosmetischen Hilfsstoffen zur Herstellung eines Präparates zur Erhöhung der Mikrozirkulation, der lokalen Regelung der Mikrozirkulation und der Immunabwehr der Haut auf Werte, die wenigstens 15 % über den Werten liegen, die ein Vergleichspräparat ohne Zusatz des Jadesteins erreicht, das dem erfindungsgemäßen Präparat entspricht und die gleiche Menge hartmagnetische Einkristalle enthält.

## Claims

1. A cosmetic and dermatological agent with magnetic particles comprising a content of 0.0001 to 2 wt. % of magnetically hard particles, selected from the group consisting of barium hexaferrite single crystals, strontium hexaferrite single crystals, samarium-cobalt particles (SmCo) and neodymium-iron-boron particles (Nd₂Fe₁₄B), the particle size ranging between 80 and 550 nm in each case, and the particles' coercive force ranging from 80.000 to 1.600.000 A/m;
and 0.0001 to 0.05 wt. % of a ground jade stone the particle size of which ranges between 50 and 95 nm;
together with cosmetic or dermatological auxiliary and carrier substances up to 100 wt. %.

2. Agent according to claim 1, **characterized in that** the magnetically hard particles are incorporated in liposomes, asymmetric lamellar aggregates or mixtures thereof in a gel.

3. Agent according to claim 1, **characterized in that** additionally asymmetric lamellar aggregates, loaded with oxygen, are contained.

4. Agent according to claim 1, **characterized in that** the share of jade stone is in the rage of 0.002 to 0.02 wt. %.

5. Agent according to claim 1, **characterized in that** it further contains 0.1 to 10 wt. % of a cosmetically acceptable solid electret with a particle size of 0.05 to 100 µm, said electret having an induced permanent dipole moment and a permanent electric field whith a field strength of 500 to 10⁷ Vm⁻¹.

6. Agent according to claim 5, **characterized in that** the electret is polytetrafluoroethylene (PTFE), fluoroethylene-propylene, polyvinylidene fluoride, amorphous fluoropolymer, tourmaline or a mixture thereof, preferably PTFE.

7. Agent according to claim 1, **characterized in that** it contains a mixture of jade and malachite.

8. A method for producing a cosmetic or dermatological agent with magnetic particles according to claim 1, **characterized in that** a mixture of magentically hard particles, jade particles, one or several fluorocarbon(s), one or several phospholipid(s), water, one or serveral monovalent and polyvalent alcohols and a gel-forming substance is prepared, and the gel obtained is mixed with further cosmetic auxiliary, carrier or active substances or mixtures thereof at a temperature ranging between 28 and 42°C, without increasing the temperature above 42°C.

9. Topical use of a mixture of magnetically hard single crystals of barium hexaferrite or strontium hexaferrite with a particle size of 50-550 nm, the content being 0.001 to 2 wt. % and the particles' coercive force ranging from 80.000 to 1.600.000 A/m, together with ground jade stone with a particle size of 50-95 nm, jade making up 0.0001 to 0.05 wt. %, and cosmetic auxiliary substances for the production of a preparation for increasing microcirculation, the local regulation of microcirculation and the immune defence to values which are at least 15 % above those values achieved by a comparative preparation that corresponds to the preparation of the invention containing the same amount of magnetically hard single crystals, without addition of jade stone.

## Revendications

1. Agent cosmétique et dermatologique comportant des particules magnétiques, ayant une teneur de 0,0001 à 2 % en poids de particules magnétiques dures, qui sont choisies dans le groupe comprenant les monocristaux d'hexaferrite de baryum, les monocristaux d'hexaferrite de strontium, les particules de samarium-cobalt (SmCo) et les particules de néodyme-fer-bore (Nd₂Fe₁₄B) ayant respectivement une taille de particules dans une plage de 80 à 550 nm, et une force coercitive des particules dans une plage de 80.000 à 1.600.000 A/m,
et 0,0001 à 0,5 % en poids de pierre de jade broyée
ayant une taille de particules dans une plage de 50 à 95 nm,
ensemble avec des excipients et des véhicules cosmétiques ou dermatologiques pour compléterjusqu'à 100 % en poids.

2. Agent selon la revendication 1, **caractérisé en ce que** les particules magnétiques dures sont dispersées dans des liposomes, des agrégats lamellaires asymétriques ou des mélanges d'entre eux dans un gel.

3. Agent selon la revendication 1, **caractérisé en ce qu'**il contient en plus des agrégats lamellaires asymétriques, chargés d'oxygène.

4. Agent selon la revendication 1, **caractérisé en ce que** la proportion en pierre de jade est dans une gamme de 0,002 à 0,02 % en poids.

5. Agent selon la revendication 1, **caractérisé en ce qu'**il a en plus une teneur de 0,1 à 10 % en poids d'une matière solide d'électret acceptable sur un plan cosmétique ayant une granulométrie de 0,05 à 100 µm, moyennant quoi l'électret a un moment dipolaire induit/permanent et a un champ électrique permanent d'une intensité de 500 à 10⁷ Vm⁻¹.

6. Agent selon la revendication 5, **caractérisé en ce que** l'électret est du polytétrafluoroéthylène (PTFE), du fluoroéthylène-propylène, du fluorure de polyvinylidène, un polymère fluoré amorphe, de la tourmaline ou un mélange de ceux-ci, de préférence du PTFE.

7. Agent selon la revendication 1, **caractérisé en ce qu'**il contient un mélange de jade et de malachite.

8. Procédé de préparation d'un agent cosmétique ou dermatologique comportant des particules magnétiques selon la revendication 1,
**caractérisé en ce qu'**on prépare un mélange de particules magnétiques dures, de particules de jade, d'un ou plusieurs fluorocarbone(s), d'un ou plusieurs phospholipide(s), d'eau, d'un ou plusieurs alcools monovalents ou polyvalents et d'un gélifiant et on mélange le gel obtenu à une température dans une plage de 28 à 42°C avec d'autres agents auxiliaires, excipients ou agents actifs ou des mélanges d'entre eux, sans que la température ne dépasse 42°C.

9. Utilisation topique d'un mélange de monocristaux magnétiques durs d'hexaferrite de baryum ou de strontium d'une granulométrie de 50 à 550 nm, en une proportion de 0,001 à 2 % en poids, et ayant une force coercitive des particules dans une plage de 80.000 à 1.600 000 A/m, ensemble avec de la pierre de jade broyée d'une granulométrie de 50 à 95 nm, la proportion de jade étant de 0,0001 à 0,05 % en poids, et d'agents auxiliaires cosmétiques pour préparer une préparation destinée à augmenter la microcirculation, la régulation locale de la microcirculation et la défense immunitaire de la peau à des valeurs qui sont au moins supérieures de 15 % à la valeur obtenue avec une préparation comparable à une préparation selon l'invention et contenant la même quantité de monocristaux magnétiques durs, mais sans ajout de pierre de jade.
